# EUROPEAN PATENT APPLICATION

(11) **EP 2 074 974 A1**
(43) Date of publication of application: **01.07.2009**
(21) Application number: 07025234.1
(22) Date of filing: 28.12.2007
(51) Int. Cl.: A61F 13/08, A47G 25/90, A61F 5/01

(54) **Auxilliary methods for applying compression devices and related apparatuses**

(71) Applicant: Mnemoscience GmbH, 52531 Uebach-Palenberg (DE)
(72) Inventor: Komoll, Torsten, 4700 Eupen (BE); Beerhalter, Ida, 65203 Wiesbaden (DE)
(74) Representative: Grünecker, Kinkeldey, Stockmair & Schwanhäusser Anwaltssozietät

(57) **Abstract**

The invention relates to a method to facilitate the procedure slipping elastane fiber-containing devices, which are designed to exert mechanical forces, over a human or animal body, a plant, or an object, **characterized in that** the device is pulled over an apparatus that is designed to retain said device in a widened configuration, and thereafter cooling the apparatus together with the pulled over device to a temperature that is below the temperature of the human or animal body, plant or object, respectively, and is low enough to fix the widened conformation by a shape memory effect of the elastane fibers, as well as to apparatuses, devices and fibers usable in the method of the invention.

## Description

### BACKGROUND OF THE INVENTION

### 1. Field of the Invention

The invention relates to a method to facilitate the procedure of slipping elastane fiber -containing devices, which are designed to exert mechanical forces, over a human or animal body, a plant, or an object; as well as to apparatuses, devices and fibers usable in this method.

### 2. Description of the Related Art

Elastomeric fibers are well known and can be made from natural or synthetic polymeric materials. Currently, these fibers are made primarily from polyisoprenes (natural) rubber or segmented polyurethanes, and to a lesser extend from segmented polyesters. In the United States, the generic designation "spandex" has been given to a manufactured fiber in which the fiber-forming substance is a long-chain synthetic polymer consisting of at least 85 weight-% of a segmented polyurethane; in Europe the equivalent term "elastane" is commonly used. Known manufacturers of elastanes are Hyosung, Invista, Bayer, and Asahi, and particularly known trade names for elastanes are Creora^{®}, Lycra^{®}, Dorlastan^{®} and Roica^{®}.

Elastanes are used in a magnitude of applications(e.g., as sportswear), and in general for applications where an elastic material is needed, such as to exert compression forces to the human or animal body, plant or object. Very high compression forces are needed, for example, for medical devices such as compression stockings that may be used as a prophylaxis or therapy against varicose veins or thrombosis.

Due to the very high forces that are necessary, in particular in medical applications, it is usually very difficult and inconvenient to apply (i.e., "pull over") such a device, and often cannot be done by the patient himself, in particular if the patient is old or weakened, and can be difficult or time consuming to apply even for personal in hospitals, health resorts or ambulant services.

To overcome this problem, coatings or extra filaments enveloping the elastane fiber have been proposed that exhibit a kind of anti-friction effect. Although helpful, they do not reduce the mechanical forces to widen the devices. Consequently, it is still difficult to pull them over. In addition, such means are an extra expenditure that makes the devices more expensive.

In addition, several apparatuses or embodiments have been proposed to help slipping into or applying such medical devices as described in, for example, German applications DE 102004047248 A1, and DE 10315870 A1; German utility models DE 20309726 U1, DE 29816222 U1, DE 199309511 U1, and DE 29708855 U1, European patents EP 1104275 B1, EP 1059907 B1, and EP 1059899 B1; and in U.S. Patent No. 6,523,729 B1. Although such apparatuses help faclilitate applying elastane fiber -containing compression devices, their handling is still dissatisfactory, as they also do not decrease the compression forces of the elastic material. Consequently, are exhausting and time consuming for the user to apply such apparatuses, in particular if very tight devices have to be used.

As can be seen from the foregoing, a high development effort has already been done and is still ongoing to simplify the procedure to apply compression devices, but nevertheless the solutions found so far are still dissatisfactory.

It was therefore an object of the invention to provide a method to facilitate the procedure of applying an elastane fiber -containing device, wherein such device is designed to exert mechanical forces to the human or animal body, a plant, or an object; as well as devices, apparatuses and elastane fibers to be used in said method.

It was another object of the invention that such a method should be feasible and inexpensive, and preferably should only need simple to use apparatuses and optionally appliances that are commonly present in households, clinics, health resorts or the like.

### SUMMARY OF THE INVENTION

The invention solves the above objects by providing a method to facilitate the procedure of slipping elastane fiber -containing devices, which are designed to exert mechanical forces, over at least a part of a human or animal body, a plant, or an object; as well as by providing apparatuses, devices and fibers usable in this method. Embodiments of the invention are described in claims 1 to 10 as well as in the following specification. In embodiments the present invention provides the following items:
1. A method to facilitate the procedure of slipping an elastane fiber -containing device, which is designed to exert mechanical forces, over at least one of a human body part, an animal body part, a plant part, and at least a part of an object comprising, (i) pulling said elastane fiber -containing device over an apparatus that is designed to retain said elastane fiber -containing device in a widened configuration; (ii) cooling the apparatus together with the elastane fiber -containing device to a temperature that is below the temperature of the at least one human body part, animal body part, plant and object, wherein said temperature is sufficiently low enough to fix the widened conformation of said elastane fiber -containing device by a shape memory effect of the elastane fibers.
2. The method according to item 1, wherein the apparatus together with the elastane fiber -containing device is cooled to a temperature of approximately - 25 °C or higher, and wherein the elastane fiber is able to fix the widened conformation by a shape memory effect at said temperature of approximately - 25 °C or higher.
3. The method according to item 2, wherein the apparatus together with the elastane fiber -containing device is cooled to a temperature of approximately 6 °C or higher, and wherein the elastane fiber is able to fix the widened conformation by a shape memory effect at said temperature of approximately 6 °C or higher.
4. The method according to any of the preceding items, wherein the elastane fiber - containing device is at least one of a garment, a wrap, a bandage, a corset and an orthosis, and wherein the elastane fiber-containing device is designed to exert mechanical forces on at least one of a human body part, an animal body part, a plant and an object.
5. The method according to item 4, wherein the device is a compression stocking.
6. An apparatus to be used in the method of any of the preceding items to retain the elastane fiber -containing device in a widened configuration, wherein the apparatus is appropriate for the given application and its stability is sufficient to keep the elastane fiber-containing device in its widened form while cooling.
7. An elastane fiber -containing device that is designed to exert mechanical forces on at least one of a human body part, an animal body part, a plant and an object usable in the method of any of items 1 to 5.
8. Use of the apparatus according to item 6 to produce the elastane fiber -containing device of item 7.
9. An elastane fiber usable for the device of item 7.
10. Use of an elastane fiber according to item 9 to produce a device according to item 7.

### DETAILED DESCRIPTION OF THE INVENTION

One aspect of the invention is a method to facilitate the procedure of slipping elastane fiber -containing devices, which are designed to exert mechanical forces, over a human or animal body, a plant, or an object, characterized in that the device is pulled over an apparatus, that is designed to retain said device in a widened configuration, and thereafter cooling the apparatus together with the pulled over device to a temperature that is below the temperature of the human or animal body, plant or object, respectively, and is low enough to fix the widened conformation by a shape memory effect of the elastane fibers.

Elastane fibers are usually composed of segmented polyurethanes, having rigid "hard" segments that provide recovery after stretching back to the fiber's original dimension, and flexible "soft" segments usually made of long polyether or polyester sections that are liquid at the temperature or use, and therefore flow past each other and allow the fiber to stretch by the effect of an external mechanical force and to recover, if that force is released. At very low temperatures (of typically approximately - 40° C or lower), the soft segments freeze usually to an amorphous state, in which they are completely rigid and brittle and cannot noteworthy be widened without break. This temperature, at which the soft segments freeze is also called the glass transition temperature of the material (Tg).

It was surprisingly found in the context of the invention that some elastanes have the ability to be fixed in a conformation at temperatures higher than the above specified Tg, but lower than the temperature at use, and that the fixity that can thereby be achieved is high enough to stabilize (fix) a widened form of compression devices including said elastanes. It was discovered, that for example in the case of compression stockings the stabilized widened form can easily be pulled over and that its handling is comparable to that of usual nylon stockings. In addition, it has been observed that despite the considerable fixity in the widened state, such devices are still flexible enough to be additionally widened or bend, as is unavoidable when pulling over such devices, and that the fibers do not break in the slip-over procedure while they are cold. Therefore, the compression properties of such devices when they have reached their usual temperature of use, are little or not negatively effected when using the method of the invention.

The elastanes useful for the method of the invention can be produced by synthetic methods known in the art. The essential elastane selection criterion is that their temperature dependent behavior be determined by DSC measurements as described in the examples or by thermomechanical analysis. Such a DSC measurement should identify a transition temperature in the temperature region to which the device is to be cooled for fixation of the widened form, or above this temperature. The elastanes selected in this way can then be tested by standard experiments to find out which of them are particularly preferred for the intended given application using the method of the invention (i.e., which have a sufficient elasticity and fixity in the stabilized widened conformation and which have of course also suitable properties necessary for the intended use and the corresponding temperature of use). Specific selection criteria for the elastanes of the invention are explicitly disclosed in the examples, and the same considerations also hold for values (peaks) taken from thermomechanical analysis, that also characterize the transition in the material that enables the fixation in the cooled state.

Particularly preferred elastanes of the invention are those with polyether soft segments.

In a preferred embodiment of the invention, the apparatus together with the pulled over device is cooled to a temperature that can be achieved with usual freezing machines, in particular with domestic freezers or even refrigerators, as this facilitates the handling and does not depend on expensive special equipment bought only for the purpose of the invention. Therefore, the widened device preferably is cooled to a temperature of approximately - 25° C or higher, in particular 0° C or higher, and particularly preferred 6° C or higher. Even temperatures near or at room temperature may be sufficient to fix the widened conformation in the method of the invention.

For the method of the invention, it is necessary that the device includes at least one type of an elastane fiber that is selected as described above and in the examples.

The device of the invention can be any device that includes at least one type of elastane fiber, and that has to be slipped over a part of a living body or an object. Such devices can be produced by the methods known in the art. Preferably, the device of the invention is a garment, wrap, bandage, corset, or orthosis, and is designed to exert mechanical forces to the human or animal body. A particularly preferred device is a compression stocking.

The apparatus to be used in the method of the invention to retain the device in a widened configuration, should have a form that is appropriate for the given application and its stability should be sufficient to keep the device in its widened form while it is cooled down. The apparatus of the invention can easily be designed and produced by known means and methods to be suitable for a given application. In this regard, the apparatuses described in the above references (see paragraph [0006]), each of which is included herein by reference in their entirety, can be used alone or in combination with other appliances as an apparatus of the invention.

In most cases it is more convenient to pull the device of the invention over the apparatus of the invention at a higher temperature than the temperature to which it is cooled to fix the widened form. Even when cooled, however, the elasticity of the device is usually high enough to be pulled over the apparatus.

The procedure to pull the device over the apparatus can in most cases be done manually, but a ready-to-use combination of widening apparatus and elastic device can also be mass produced and utlized (e.g., as a pre assembled product, that can be stored at room temperature and may only be cooled prior to use). Such a product could be a single-use product or a service could be established to renew the ready-to-use configuration.

In an embodiment of the invention that is in particular suitable for use when traveling, the flask could be filled with a mixture of salt, water, and ice, or only with water and ice, if an appropriate elastane is used, to cool down the device without the need of an own freezer or refrigerator, as both salt and/or ice usually are readily available.

It will be apparent to those skilled in the art that various modifications and variations can be made in the present invention and specific examples provided herein without departing from the spirit or scope of the invention. Thus, it is intended that the present invention covers the modifications and variations of this invention that come within the scope of any claims and their equivalents.

The following examples are for illustrative purposes only and are not intended, nor should they be interpreted to, limit the scope of the invention.

### EXAMPLES

**Method to determine the temperature dependent behavior of elastanes and to select elastanes suitable for the present invention**

The thermal properties of elastanes were evaluated by differential scanning calorimetry (DSC) with a Mettle Toledo DSC 822 equipment using 5 to 10 mg of material and heating between -100 to 150° C with a heating rate of 10 K / minute and a cooling rate of 10 K / minute. Two scans were recorded and the data were taken from the second heating scan. The peak related to a shape transition had a certain temperature range, that was analyzed by measuring the temperature, at which the transition begins (**Peak(1,2)** Tm, onset), the temperature at the maximum of the peak (**Peak(1,2) Tm, peak)**, and the temperature at which the transition is completed (**Peak(1,2) Tm, end). Tm, onset** was obtained mathematically from the measurement at the point, at which the tangent through the inflection point at the low-temperature side of the peak subtends the straight line best fitted to be the baseline underlying the peak. In cases where no single inflection point could be indentified, but a region with an approximately constant gradient, a straight line through said region was used instead of the tangent. **Tm, end** is obtained accordingly at the high temperature side of the peak.

Elastanes of the invention are selected so that their **Tm, end** is at least approximately identical, and preferably higher, than the temperature that is intended to be used to fix the widened configuration (**T_{fix}**), in particular so that their **Tm, peak** is at least identical, and preferably higher, than **T_{fix}**, and if possible, so that also their **Tm, onset** is approximately identical, or higher than **T_{fix}. T_{fix}** is only limited at the lower temperature side by the low **Tg** of the elastanes of the invention, at which they become rigid.

Elastanes of the invention are in addition selected, so that their **Tm, onset** is at least approximately identical, and preferably lower, than the temperature that is usually present at the intended use after slipping over the device (**Tᵤₛₑ**), in particular so that their **Tm, peak** is at least identical, and preferably lower, than **Tᵤₛₑ**, and in a particularly preferred embodiment of the invention, that also their **Tm, end** is approximately identical, and preferably lower than **Tᵤₛₑ**.

**Production of compression stockings and evaluation of the method of the invention.**

Compression stockings were woven into a fabric using the elastane fiber (yarn) Dorlastan® dtex 235 V820 (polyether based). Said elastane fiber had a Tm, onset of approximately -8° C, a **Tm, peak** of approximately 6° C, and a **Tm, end** of approximately 13° C, and was therefore well suited for the current tests, wherein the temperature of use (Tᵤₛₑ) was approximately 35° C (i.e., the temperature on the surface of a human leg and foot). The stockings were open at the toe region and were taken for the tests that are described in the following. For tests 1, 2 and 4 there was inserted a simple conical flask with an appropriate dimension and placed into the ankle region of the stocking, as this region is most critical for the slip-over behavior of the stocking. The so-widened stocking was cooled in a freezer to a temperature of approximately -20° C (**T_{fix}**).

**Test 1 (invention)**: a person slipped in the widened and cooled stocking, that still did contain the flask and pressed out the flask with its feet while slipping in.

**Test 2 (invention)**: the flask was removed out of the widened and cooled stocking, before the person did slip in.

**Test 3 (comparison)**: the person did slip in the stocking without any help or pretreatment of the stocking.

**Test 4 (comparison)**: the person did slip in the stocking that was widened with the same flask as used for the tests of the invention, but without cooling, to simulate the use of commercially available mechanical aids. As in test 1 the flask was pressed out with the feet while slipping in.

**Test 5 (comparison)**: the person did slip in the stocking that was widened with the same flask as used for the tests of the invention, but without cooling, to simulate the use of commercially available mechanical aids. As in test 2 the flask was removed out of the widened stocking, before the person did slip in. The material did fully contract immediately after removal of the flask.

**Discussion of the results**

Tests 1 and 2 of the invention demonstrated impressively the advantages of the invention. It was much easier to slip in the stocking and comparable to put on usual nylon stockings, when using the method of the invention vis-à-vis the comparison tests, where high forces were necessary to widen the stocking while slipping in. There was no felt difference in the forces needed to slip in the stockings with respect to tests 3 and 5.

Leaving the flask in the stocking in test 1 was found to be an extra help and had the additional advantageous effect, that the temperature remained low for a longer time. Without the flask it was necessary to slip in the stocking directly after taking it out of the freezer, preferably within less than one minute, to achieve the full advantage of the invention.

Although the stocking was at a temperature of -20° C in the tests of the invention, this was not felt to be unpleasant. As the stocking is rather thin, it warms up very quickly by the contact with the body and it was found, that it then exerts approximately the same compression forces to the leg as the stocking that was used without the help of the method of the invention.

Although the invention has been described and illustrated with a certain degree of particularity, it is understood that the present disclosure has been made only by way of example, and that numerous changes in the conditions and order of steps can be resorted to by those skilled in the art without departing from the spirit and scope of the invention.

## Claims

1. Method to facilitate the procedure to slip over a device that comprises elastane fibers and is designed to exert mechanical forces to at least a part of the human or animal body, a plant, or an object, **characterized in that** the device is pulled over an apparatus, that is designed to retain said device in a widened configuration, and thereafter cooling the apparatus together with the pulled over device to a temperature that is below the temperature of the human or animal body, plant or object, respectively, and is low enough to fix the widened conformation by a shape memory effect of the elastane fibers.

2. Method according to claim 1, wherein the apparatus together with the pulled over device is cooled to a temperature of approximately - 25 °C or higher, and wherein the elastane fiber is able to fix the widened conformation by a shape memory effect at said temperature.

3. Method according to claim 2, wherein the apparatus together with the pulled over device is cooled to a temperature of approximately 6 °C or higher, and wherein the elastane fiber is able to fix the widened conformation by a shape memory effect at said temperature.

4. Method according to any of the preceding claims, wherein the device is a garment, wrap, bandage, corset, or orthosis, and is designed to exert mechanical forces to the human or animal body.

5. Method according to claim 4, wherein the device is a compression stocking.

6. Apparatus to be used in the method of any of the preceding claims to retain the device in a widened configuration, **characterized in that** the form of the apparatus is appropriate for the given application and its stability is sufficient to keep the device in its widened form while it is cooled down.

7. Device that comprises elastane fibers, and is designed to exert mechanical forces to the human or animal body, a plant, or an object, usable in the method of any of claims 1 to 5.

8. Apparatus according to claim 6 with pulled over device according to claim 7.

9. Elastane fiber usable for the device of claim 7.

10. Use of an elastane fiber according to claim 9 to produce a device according to claim 7.
